# EUROPEAN PATENT SPECIFICATION

(11) **EP 4 615 636 B1**
(45) Date of publication and mention of the grant of the patent: **11.11.2026**
(21) Application number: 23776605.0
(22) Date of filing: 21.09.2023
(51) Int. Cl.: A61L 2/22, A61L 9/14, B05B 7/04

(54) **SUPERSONIC NOZZLE FOR DECONTAMINATION AND/OR DISINFECTION**
ÜBERSCHALLDÜSE ZUR DEKONTAMINATION UND/ODER DESINFEKTION
BUSE SUPERSONIQUE POUR LA DÉCONTAMINATION ET/OU LA DÉSINFECTION

(30) Priority: 08.11.2022 ES 202230959; 18.11.2022 US 202217990159
(43) Date of publication of application: 17.09.2025
(73) Proprietor: COUNTERFOG S.L., 28341 Valdemoro Madrid (ES)
(72) Inventor: PEREZ DEL ALAMO, Francisco Javier, 28341 Valdemoro Madrid (ES); GARCIA CASARRUBIOS, Juan Sanchez, 28341 Valdemoro Madrid (ES)
(74) Representative: Lorente Berges, Ana
(86) International application number: PCT/EP2023/076084
(87) International publication number: WO 2024/099631

(56) References cited:
- EP-A1- 3 395 449
- US-A1- 2001 043 888

## Description

### FIELD

The present invention relates to apparatus and methods used for decontamination and disinfection purposes.

### BACKGROUND

Atomization of a liquid into fine droplets has a large number of industrial applications including, for instance, moisting, painting, injecting, cleaning, coating, lubrication, dust control, humidification, fire protection, cooling solids, gas cooling, washing and conditioning, covering and for decontaminating, capturing, or sampling air-borne particles -including for example smoke, viruses, and spores.

Droplet size, geometry and dynamics of flow and spatial distribution are essential for particular purposes like decontamination of micron and submicron-sized air-borne particles or for the sampling of aerosols. For example U.S. Patent Application Serial No. 17/687,066 discloses a method and apparatus for capturing and sampling air-borne particles using a jet of fog. Also, as an example air decontamination based on the use of jets of fog generated with nozzles was described by Perez-Diaz et al. in 2019 "Decontamination of Diesel particles from air by using the Counterfog® system. Air Quality, Atmosphere & Health. 12. 10.1007/s11869-018-00656-7". In this particular application the size of the droplets conditions the effectiveness. Only droplets smaller than the air-borne particles can collapse and aggregate to them. Therefore, to remove the finest airborne particles smaller than a micron, it is necessary to generate jets of fog made of droplets smaller than or of the order of airborne particles . For instance, SARS-Cov-2 has a diameter around 125 nm and Anthrax spores have a diameter of around 800 nm.

A variety of nozzle designs and sizes are available to produce fine droplets. In direct pressure nozzles, the fluid is broken (atomized) into droplets by impact on a surface or by the high shear force caused by the fluid passing through a shaped orifice, as in U.S. Publication No. 2006/0196967 A1. The energy required for atomization comes from the energy of the fluid itself.

By contrast, atomizing nozzles based on the mixture of two fluids, air (or other gas) and liquid, represent a more efficient option since their operation is based on the speed and pressure gradient between the gas at high speed and an injected or aspirated liquid (Venturi effect) at low speed and low pressure. These different relative speeds between gas and liquid phases are key for atomizing viscous media at low pressures. This means that the energy required for atomization is independent to the fluid pressure, allowing fine atomization at low fluid pressures.

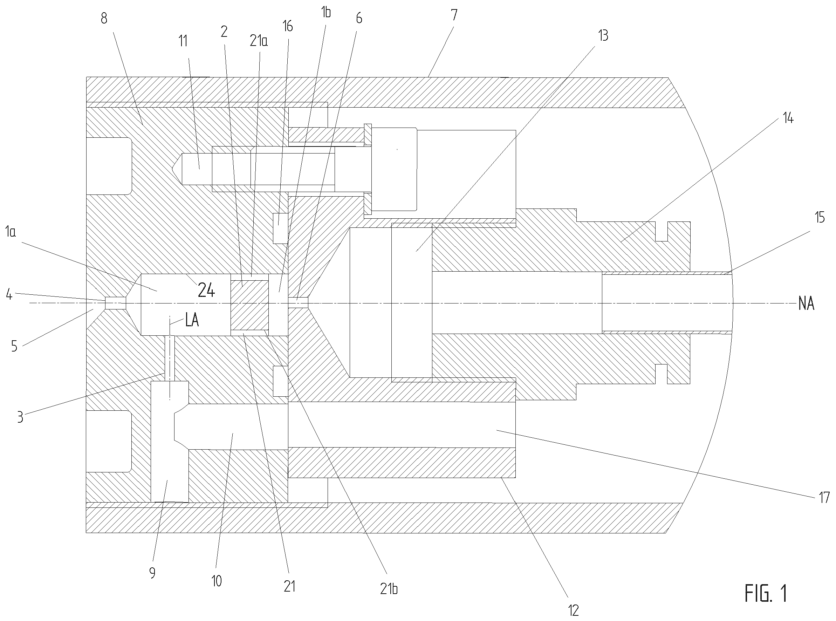

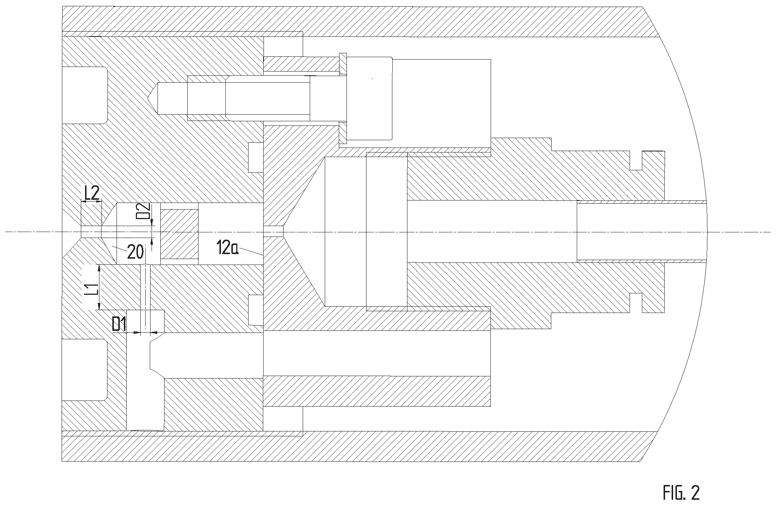

The use of slotted disks as internal parts in atomizing nozzles is well known. Examples are found in U.S. Patent Nos. 5,692,682 and 7,611,080 and in U.S. Publication Nos. 2003/0146301 A1 and US2015/0028132 A1. All of them with the function of inducing a spin or tangential component added to the axial velocity of a liquid, a gas or a mixture of both. These slotted disks or swirls need a relatively large diameter to add a tangential component to the fluid speed not negligible compared to the axial one -always in subsonic regime. In this sense EP3395449B1 describes as well two types of atomizing nozzles using two chambers separated by a "swirl disk" with slanted fluid passages. The first type (Figs 1 and 2 therein) is provided with a passage so-called "single vertical conduct 63" therein to supply liquid and a so-called "slanted conduct 64" therein to supply a second fluid -that may be air according to the cited document. The convergence -due to the slant angle- of the flows of the liquid and the air into a point in the so-called "second mixing chamber" produces an atomization. In this type the "first mixing chamber" (in a rear position with respect to the nozzle outlet) is just used to supply air but -in spite of its name- no mixing or atomization occurs in such a chamber.

The second type described in EP3395449B1 (Fig. 3 therein) is provided with a "swirl disc" provided with a plurality of "slanted lateral conducts". The liquid flows from a rear chamber to a front chamber simultaneously through all the "slanted lateral conducts". Their slant -being the same for all the "slanted lateral conducts"- causes swirling to the liquid reaching the front chamber improving atomization in the front chamber. Again, mixing and atomization occur just in the front chamber. These devices, additionally, do not pay attention to the outlet geometry, making it just as short as possible to let the mist already generated in the front chamber to escape as soon as possible and with minimal pressure losses (as it is intuitive to the expert in the field for a subsonic flow of air). Moreover, most mist generating nozzles do not even aim to generate a "jet" of fog, but just to slowly and gently discharge mist into the environment.

Pressure, geometry and mass-flows are critical parameters for the results of a liquid-gas mixing that can produce bubles, foam, emulsions or a full range of sprays, fog or mist with sizes ranging from milimeters to microns. For instance, documents JP2004298694, JP2006187752 and KR 20210145451A teach the use of high pressure liquid and compressed air to provide a water-air mixture (foam) jet for car wash or similar applications. It is evident that the higher the pressure it needs the more inconvenient and expensive is a device. These three documents use a relatively long outlet pipe for focalization of the foam flow. Note that a subsonic foam flow in a pipe will get the typical viscous speed profile with a maximum value at the axis of the pipe that helps to generate a cohesive foam jet. In any case these three mentioned devices were never intended to provide a fog or a mist with fine liquid droplets.

Producing fine droplets from liquids by mixing gas and liquid in a nozzle involves complex phenomena which are not yet fully understood. This lack of knowledge has prevented an efficient optimization of droplet sizes and output velocity. Uniform high-speed aerosol patterns are not achievable with the current state of the art. In general, current devices are able to produce small droplets only at low discharge speed. Therefore, they proved to suffer from drift and, consequently, are ineffective for the majority of applications.

In recent years, improvements in atomization efficiency have been proposed through the use of Laval geometries as Zhang T et al. teach in "Supersonic antigravity aerodynamic atomization dusting nozzle based on the Laval nozzle and probe jet. J Braz Soc Mech Sci Eng 2020; 42: 335". This dedicated profile (so called Laval profile), widely used in nozzles for rocket engines, accelerates from subsonic to supersonic regime a gas flow through a throat that communicates the convergent inlet to the divergent outlet. Its use presents serious limitations because liquid cannot reach the supersonic region and cannot combine with the supersonic air in an optimum way as Cavaliere P. explained in 2015 in "Cold spray coating technology for metallic components repairing. Through-life engineering services. Springer International Publishing, Berlin. DOI:10.1007/978-3-319-12111-6_11". Consequently, the air-water mixing is only performed in the divergent outlet (expansion process) and always outside the nozzle. Any expansion process implies a temperature drop, any liquid, under these conditions, increases its surface tension so the atomization becomes inefficient.

F.M. White (1979 Fluid Mechanics, Mc Graw Hill) teaches how subsonic and supersonic gas flow regimes behave essentially in a different way. Supersonic regime is particularly counterintuitive and not easy to understand and replicate. Speed increases through a convergent duct for a subsonic flow, but it decreases for a supersonic one. Speed decreases through a divergent duct for a subsonic flow while it increases for a supersonic flow. Therefore, a specific geometry produces essentially different effects depending on the kind of regime (subsonic or supersonic). F.M. White teaches as well how a transition from subsonic to supersonic can occur when the fluid passes through a throat or a convergent-divergent duct. White says that this transition cannot appear in a divergent-convergent duct where the subsonic or supersonic characteristic would remain unchanged. However, counterintuitively again, friction with the walls of the pipe can increase the Mach number and even generate a transition to supersonic even in a constant section duct due to the combination of heat transfer and pressure drop that causes expansion of the fluid and subsequent acceleration (https://engineering.stackexchange.com/questions/35603/why-dont-all-gas-pipe-flows-end-up-at-sonic-speed published on May 29, 2020 and accesed on Sep 12. 2023). However, it is evident that speed cannot be increased indefinitely and therefore a too long duct may also prevent supersonic flow to occur.

Potter and Wigget (2010, *Mechanics of Fluids,* 3rd SI ed., Cengage) teach that the condition to provide a supersonic gas flow through a de Laval geometry pipe or through an orifice is that $\frac{{P}_{1}}{{P}_{2}} \geq {\left(\frac{2}{γ + 1}\right)}^{\frac{γ}{1 - γ}}$ where γ is the heat capacity ratio of the gas, P₁ is the upstream pressure supplied to the inlet of the pipe, P₂ is the downstream pressure . For the case of dry air, the condition for supersonic flow is that the pressure ratio overpasses the following critical value: $\frac{{P}_{1}}{{P}_{2}} \geq \frac{1}{0.587} = 1.704$. In the case of a discharge to an environment at 1 bar this condition turns to be just P₁≥ 1.704 bar.

They also teach that in the supersonic regime the mass flow *ṁ* becomes choked and its value depends on the cross-sectional area A, among other parameters like the dimensionless discharge coefficient C_{d} or ρ₁ real gas density at pressure P₁ and temperature T₁ at the inlet: ${m}^{⋅} = {C}_{d} A \sqrt{{γρ}_{1} {P}_{1} {\left(\frac{2}{γ + 1}\right)}^{\frac{γ + 1}{γ - 1}}}$

If upstream pressure P₁ increases then the mass flow increases as well (and the upstream density increases too). According to this expression, the choked mass flow of an ideal gas does not depend on downstream pressure P₂ -provided the above inequality is fulfilled-. However, Cunningham taught that the discharge of real gases through thin-plate orifices seems to depend slightly on the downstream pressure or equivalently the choked flow will not occur across a standard, thin, square-edge orifices (Cunningham, R.G., "Orifice Meters with Supercritical Compressible Flow" Transactions of the ASME, Vol. 73, pp. 625-638, 1951). Therefore, these analytical expressions may be a useful rough guide to determine the occurrence of supersonic flows and estimate their characteristics but require either experimental or computational verification. Eventually, accurate well-known computational models can provide a good reliable approach to research the flow regimes in a particular geometry under particular pressure and therefore used to find the particular critical pressure for supersonic transition for a particular geometrical configuration of a duct, as for example it is described by Fedoseyev (Finite element method stabilization for supersonic flows with flux correction transport method, AIP Conf. Proc. 2302, 120003 , 2020).

There are alternatives to mitigate the above mentioned constraint for atomization in supersonic regime by attempting partial pre-atomization prior to convergent entry before reaching supersonic speeds, for example, by using annular/coaxial geometries in the liquid and air discharge, as disclosed in U.S. Patent Nos. 5,520,331 and 3,534,909. Another alternative is the use of ultrasonic vibrating pins as Hong et al. explained in 2011 in "Resonant behaviors of ultrasonic gas atomization nozzle with zero mass-flux jet actuator. J Shanghai Univ (English Edition) 15(3):166-172". However, as the pre-atomization alternatives are not perfect, the inefficient droplet size distribution with large droplets generates strong decelerations and a non-uniform discharge flow both in speed and spatial distribution, which in any case, does not reach nanometer sizes and is too large to be considered as an aerosol distribution and additionally periodically clugging the nozzle outlet. All these three last cited references agree that this causes undesired pulsated and non-uniforme discharge.

The results of these partial solutions are not efficient enough due to the complexity of the solution itself, the complex manufacturing of the nozzle as Cai et al. teach in "Optimum end milling tool path and machining parameters for micro-Laval nozzle manufacturing. Proceedings of the Institution of Mechanical Engineers, Part B: Journal of Engineering Manufacture. 231. 10.1177/0954405415608601" or the additional energy consumption in case of using ultrasonic pre-atomization. The generation of aerosols at high discharge speeds is still a real challenge today.

### SUMMARY

Disclosed herein are nozzles that solve the problems discussed above by means of an efficient mixing of a gas (e.g. air) and a liquid (e.g. water) achieved by means of first, second and third atomization stages. The first atomization stage occurs in a rear sub-chamber of the nozzle and the second atomization stage occurs in a front sub-chamber of the nozzle. The front and rear sub-chambers are separated by a dividing member. The dividing member includes two or more fluid passages that extend along an entire axial length of the dividing member, the two or more fluid passages provide fluid communication between the front and rear sub-chambers. According to one implementation, in the first atomization stage the rear sub-chamber is used to mix a low-pressure axial water stream with air proceeding from the front sub-chamber creating a mist/fog/aerosol (e.g. water droplets suspended in air) distribution in the rear sub-chamber. The air proceeding from the front sub-chamber enters the rear sub-chamber through one or more first fluid passages located in the dividing member. The second atomization stage involves a mist/fog generated in the rear sub-chamber being introduced into the front sub-chamber by one or more second fluid passages in the dividing member and further atomizing the liquid in the incoming mist/fog to create a new finer droplet distribution by use of a supersonic pressurized air flowing into the front sub-chamber. The high gradient of velocity associated to the supersonic front very efficiently breaks the liquid droplets. Finally, the fine mist/fog produced in the front sub-chamber during the second atomization stage is accelerated to supersonic speeds in the third atomization stage by means of a narrow nozzle outlet conduit having an outlet diameter that is equal to or less than one-half its length. (This avoids the need to apply Laval geometries.) As a result, a generally uniform and anti-drift supersonic expansion cone of aerosol/fog is jetted from the nozzle with an ability to suppress chemical, biological, nuclear, and radiological (CBRN) aerosol from the air external to the nozzle. "Anti-drift" meaning the supersonic aerosol expansion cone created at the outlet of the nozzle has an inertia that is not easily affected by air flow external to the nozzle.

In the examples provided herein, the dividing member is a disk. It is appreciated, however, that the dividing member need not comprise a cylindrical shape. Rectangular and other shapes are also contemplated. The dividing member need only facilitate flow between the front and rear sub-chambers as disclosed herein in order that the first, second and third atomization stages take place upon a designated flow of gas (e.g. air) and a designated flow of liquid (e.g. water) is delivered respectively to the front and rear sub-chambers.

As will be described in more detail below, according to some implementations the dividing member is a slotted disk that includes slots/grooves formed in an outer circumferential wall of the disk, the slots/grooves extending entirely across the axial length of the disk to fluidly communicate the front and rear sub-chambers with one another. In lieu of or in conjunction with the slots/grooves, the dividing member (e.g. disk) may include through holes located near a peripheral edge of the disk through which the front and rear sub-chambers communicate.

In the context of the present disclosure, "located near a peripheral edge" means the through holes are located nearer the peripheral edge of the disk than to the axial center of the disk.

According to some implementations, the axial position of the dividing member within the nozzle may be adjusted to alter the volume of the front and rear sub-chambers for the purpose of altering the jet produced at the outlet of the nozzle 100.

The use of slotted disks as internal parts in atomizing nozzles is well known, Examples are found in U.S. Patent Nos. 5,692,682 and 7,611,080 and in U.S. Publication Nos. 2003/0146301 A1 and US2015/0028132 A1. All of them with the function of inducing a spin or tangential component added to the axial velocity of a liquid, a gas or a mixture of both. These slotted disks or swirls need a relatively large diameter to add a tangential component (inertial momentum) not negligible compared to the axial one. U.S. Patent No. 7,243,861 B2 is an example application in this regard. It discloses a divergent part, a disk with a diameter taking up between 5 mm and 10 mm with passages which are oblique and/or in the form of helical portions with a "total area" "of between about 3 mm² and about 15 mm² for inducing rotation in water.

In the case of atomizing nozzles based on supersonic discharge, the use of spin-inducing slotted disks is meaningless, all induced spin is cancelled out in the acceleration process to supersonic speeds (axial component predominates). The present invention may employ a smaller diameter slotted disk than used in the present state of the art for reason that it is not required to drive a tangential component to the fluid. For example, slotted disks of less than 5 mm in diameter with helical indentations of less than 3 mm² in cross-section may be used. A main function of the slotted disk of the present invention is to modify the volume of the front and rear sub-chambers allowing interconnection between them in order to optimize the supersonic aerosol droplet size distribution discharged into the environment.

Nozzles used for decontamination or disinfection may become contaminated by contact with the contaminated environment, particularly with air. The anti-drift supersonic aerosol expansion cone created by use of the supersonic nozzles disclosed herein diminishes or eliminates the flow of contaminates into the interior of the nozzle.

These and other advantages and features will become apparent in view of the drawings and the detailed description.

### BRIEF DESCRIPTION OF THE DRAWINGS

FIG. 1 illustrates a cross-section side view of a supersonic nozzle according to one implementation.
FIG. 2 illustrates the supersonic nozzle of FIG. 1 with the dividing member located in a different axial position.
FIG. 3A is a view of a dividing member according to one implementation.
FIG. 3B is a view of a dividing member according to another implementation.
FIG. 4 illustrates example flowlines of compressed air and mist in the sub-chambers, disk slots and outlet of the supersonic nozzle of FIG. 1.

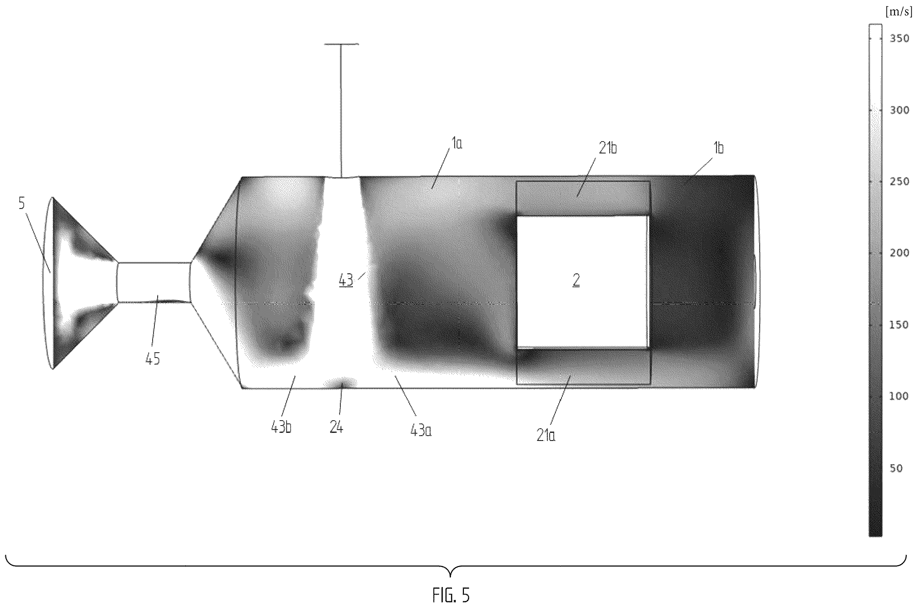

FIG. 5 is a greyscale graph illustrating an example magnitude of the fluid speed in the sub-chambers, disk slots and outlet mouth of the supersonic nozzle of FIG. 1. The scale is adjusted so that supersonic regions are approximately represented as white.
FIG. 6 is a side cross-section view of a nozzle assembly according to one implementation.

### DETAILED DESCRIPTION

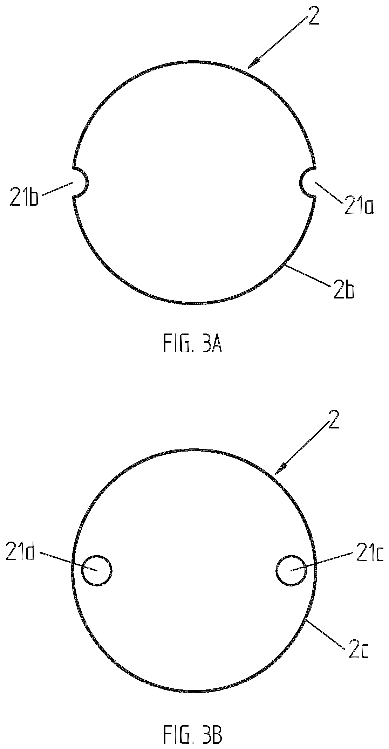

A supersonic nozzle according to one implementation comprises an atomizing chamber 1 (which may be drilled as a cylindrical hole) with a dividing member 2 inserted in it dividing the atomizing chamber into a front sub-chamber 1a and a rear sub-chamber 1b. In the examples provided herein, the dividing member 2 is a disk as shown in FIGS. 3A and 3B. As explained above, the dividing member 2 need not comprise a cylindrical shape. Rectangular and other shapes are also contemplated. The dividing member 2 need only facilitate flow between the front and rear sub-chambers 1a and 1b as disclosed herein in order that the first, second and third atomization stages take place upon a designated flow of gas (e.g. air) and a designated flow of liquid (e.g. water) being respectively delivered into the front and rear sub-chambers. In the implementation of FIG. 3A, the dividing member 2 is a slotted disk that includes slots/grooves 21a and 21b formed in an outer circumferential wall 2b of the disk, the slots/grooves extending entirely across the axial length of the disk to fluidly communicate the front and rear sub-chambers with one another. These slots/grooves 21a and 21b may be of a different flow cross section. In lieu of or in conjunction with the use of slots/grooves, the dividing member 2 (e.g. disk) may include through holes 21c and 21d located near a peripheral edge 2c of the disk 2 as shown in FIG. 3B through which the front and rear sub-chambers communicate. Again these holes 21c and 21d may be of a different flow cross section.

The front sub-chamber 1a is provided with a lateral narrow compressed air inlet conduit 3 that opens into the front sub-chamber. The air inlet conduit 3 has a diameter D1 substantially smaller than its length L1. The air inlet conduit 3 is cylindrical and has a diameter D1 that is equal to or less than half its length L1. According to some implementations D1 ranges from 0.3 mm to 1.1 mm and L1 ranges from 2.0 mm to 4.5 mm. According to some implementations, the air inlet conduit 3 has a central longitudinal axis "LA" that is arranged perpendicular to the nozzle axis "NA". According to other implementations (not shown), the air inlet conduit 3 has a central longitudinal axis "LA" that is arranged oblique to the nozzle axis "NA". The front sub-chamber 1a also fluidly communicates with a narrow outlet conduit 4 that exhaust to the environment. Like air inlet conduit 3, the narrow outlet conduit 4 also has diameter substantially smaller than its length. In addition, the diameter of the narrow outlet conduit 4 is greater than the diameter of the air inlet conduit 3. The outlet conduit 4 is cylindrical and has a diameter D2 equal to or less than one-half its length L2. The cross section of the narrow outlet conduit 4 is greater than the cross-section of the air inlet conduit 3 to cause the compressed air to have a first expansion when entering the front sub-chamber 1a and a second expansion when exiting the narrow outlet conduit 4. Moreover, air pressure is provided (typically between 4 and 12 bar) to generate first and second supersonic discharges: the first supersonic discharge occurring at the outlet of air inlet conduit 3 into the front sub-chamber 1a and the second supersonic discharge being from the front sub-chamber 1a out through the narrow outlet conduit 4. Typically pressure drops by 1 to 2 bar in the first supersonic discharge from the air inlet conduit 3 into the front sub-chamber 1a while pressure drops to almost atmospheric pressure in the second supersonic discharge from the front sub-chamber 1a out through the narrow outlet conduit 4 generating a jet of fog. According to some implementations D2 ranges from 0.5 mm to 1.5 mm and L2 ranges from 0.6 mm to 4.0 mm. According to some implementations, the ratio of D2/D1 is in a range of 0.8 to 1.4.

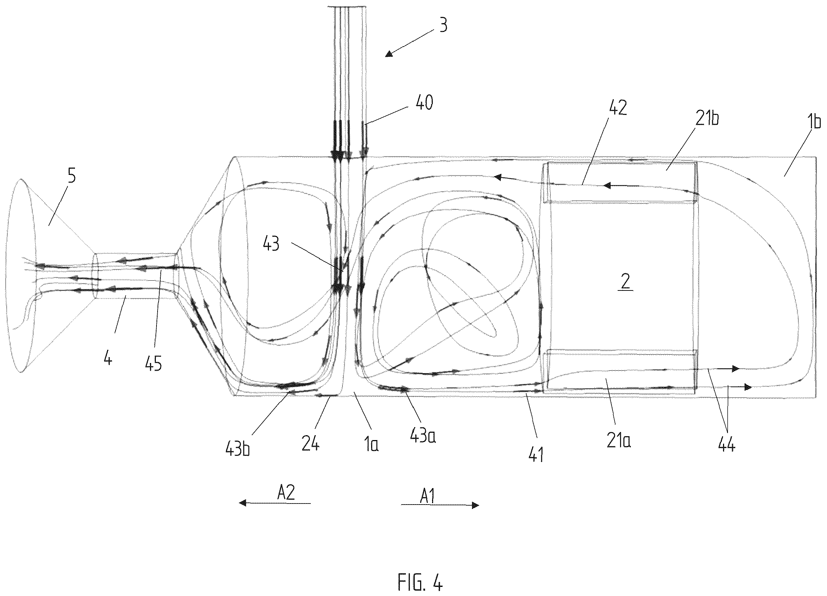

The nozzle 100 is configured such that when compressed air 40 is delivered into the front sub-chamber, the compressed air partially penetrates from the front sub-chamber 1a into the rear sub-chamber 1b through one or more first slots 21a (and/or first holes 21c) and returns to the front sub-chamber 1b back through one or more second slots 21b (and/or second holes 21d). The disk 2 includes only two slots (or two holes), a first slot 21a (or hole 21c) through which pressurized air/gas 41 passes from the front sub-chamber 1a to the rear sub-chamber and a second slot 21b (or hole 21d) through which a mist 42 generated in the rear sub-chamber 1b passes to the front sub-chamber 1a. Typically, the supersonic air flow 43 coming into the front sub-chamber from the air inlet conduit 3 collides with a wall 24 of the front sub-chamber 1a. According to some implementations, the wall 24 is located opposite the entry location of the supersonic air flow (i.e. at the outlet of air inlet conduit 3). As illustrated in FIG. 4, the collision causes a first part of the supersonic air flow 43a to be directed in a first axial direction A1 towards the disk 2 and causes a second part of the supersonic air flow 43b to be directed in a second axial direction A2 towards a front end portion of the front sub-chamber 1a. With continued reference to FIG. 4, at least a portion of the A1 directed air penetrates through the one or more first slots 21a (or one or more first holes 21c) of disk 2 into the rear sub-chamber 1b where it mixes with a liquid (e.g. water) to produce a mist that migrates towards and flows through the one or more second slots 21b (or one or more second holes 21d) into the front sub-chamber 1a.

The rear sub-chamber 1b is provided with a liquid inlet 6 supplying liquid at a pressure greater than atmospheric pressure to be atomized by the air flow 44 in the rear sub-chamber 1b. A first atomization is achieved in the rear sub-chamber 1b as the liquid is mixed with the air flow therein. The number and size of liquid droplets generated in the rear sub-chamber 1b will be conditioned by the diameter of the liquid inlet 6, the supply pressure of the liquid at the liquid inlet 6, and the amount of air flow in the rear sub-chamber 1b. According to one implementation, the diameter of the liquid droplets in the rear sub-chamber is between 0.5 to 1 mm (when atomized). The air flow in the rear sub-chamber 1b is substantially slower than the supersonic air flow in the front sub-chamber 1a and can be altered/tuned by moving the axial location of the disk 2 along a length of the chamber 1. As an example, FIGS. 1 and 2 show the disk 2 at different axial locations. The air flow can also be altered/tuned by changing the number of slots or holes, changing the geometry or size of the slots or holes, or by changing their orientation (e.g. by slanting the slots or through holes).

The mist/fog 42 generated in the rear sub-chamber 1b flows into the front sub-chamber 1a through the one or more second slots 21b (or one or more second holes 21d). This mist/fog is drawn into the front sub-chamber 1a by the supersonic air flow coming in from the air inlet conduit 3 and breaking (or more precisely by bursting) the liquid droplets into much smaller ones. This causes a second atomization of the liquid droplets that homogenizes the properties of the mist/fog 45 as it passes through the narrow nozzle outlet conduit 4, and therefore homogenizes the properties of the jet of fog produced at the nozzle outlet. This second atomization is so effective that liquid droplets can be generated at the nozzle outlet with diameters as small as a few tens of nanometers (e.g. 40 to 50 nanometers).

The nozzle may be operated in a second mode to provide larger water droplets in the mist/fog that is generated out the outlet of the nozzle 100. In instances this may be advantageous, such as when it is necessary to cover a surface with water droplets. According to one implementation, this can be accomplished by increasing the liquid pressure and flow through the liquid inlet 6 to cause a filling of the rear sub-chamber 1b with the liquid. When operating in the second mode, the liquid in the rear sub-chamber 1b flows through some or all of the plurality of slots and/or holes of the disk 2 into the front sub-chamber 1a to be atomized therein by the flow of supersonic air entering the front sub-chamber via compressed air inlet 3. According to some implementations, when the nozzle is operated in the first mode as described above, a liquid (e.g. water) is delivered through the liquid inlet 6 at a pressure of 1 to 2 bar. According to some implementations, switching to the second mode may occur by increasing the liquid/water pressure in the liquid inlet 6. According to some implementations, a switching of operation from the first mode to the second mode occurs when the liquid/water in the liquid inlet 6 reaches a pressure of between about 4 bar to about 10 bar.

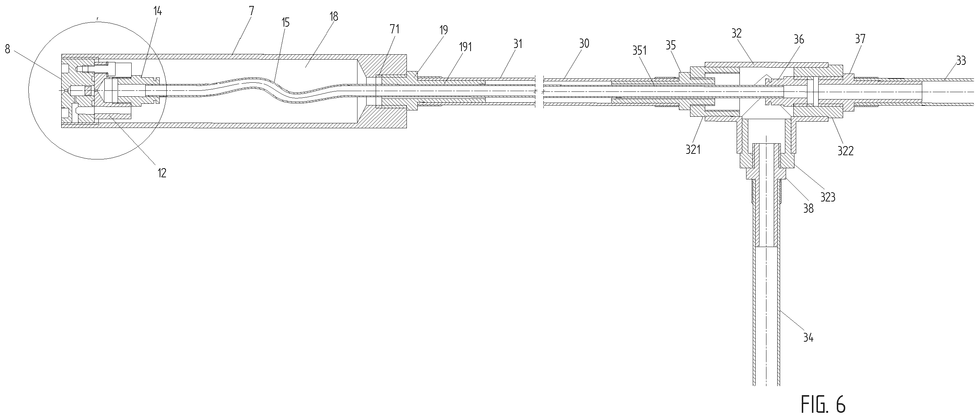

Turning now to FIG. 6, according to some implementation the supersonic nozzles disclosed and contemplated herein may reside inside a housing 7 that can be easily held and manipulated by a user. According to some implementations, the nozzle includes a plug 8 secured to a front end of housing 7. According to some implementations, the housing 7 and plug 8 are joined by a threaded connection. According to some implementations the plug 8 has a front face that is substantially flat and flush with a front border of the housing 7. This flat/flush arrangement makes it easier to clean and decontaminate the nozzle exterior surface after use. The housing 7 forms a cavity 18 through which air from the air supply line 34 (via outer conduit 31 of co-axial hose 30) supplies air to the nozzle.

According to some implementations, the front face of the plug 8 is provided with slots arranged with a hexagonal or square shape to facilitate the use of a socket wrench to tighten the plug 8 onto the housing 7.

According to some implementations, the front sub-chamber 1a is delimited by a back face of the plug 8. According to some implementations, the narrow outlet conduit 4 is disposed between an internal cylindrical or cone-shaped hole 20 and a conic outlet mouth 5. According to some implementations, the narrow outlet conduit 4 comprises a hole drilled into the front face of the plug 8 and the outlet mouth 5 and hole 20 respectively located in a front face and a back face of the plug are axially aligned. The configuration of the conic outlet mouth 5 determines the geometry of the jet of fog exiting the nozzle.

According to some implementations, a part of the nozzle includes a radial bore 9 through which compressed air is delivered to air inlet conduit 3. According to some implementations, the radial bore 9 has a cross-sectional area greater than the cross-sectional area of the air inlet conduit 3 so that the compressed air flowing through the radial bore flows sub-sonically. According to some implementations, each of the radial bore 9 and air inlet conduit 3 is cylindrical with the radial bore having a diameter greater than the diameter of the air inlet conduit. According to one implementation the air inlet conduit 3 has a diameter of 0.65 mm and produces approximately a 1,500 meters/second supersonic flow when compressed air is supplied to the air inlet conduit at 10 bar. According to such an implementation, when the diameter of the radial bore 9 is 2.5 mm, the compressed air flows sub-sonically through the radial bore at a speed of less than 100 meters/seconds.

According to some implementations, an axial air passage 10 is also drilled the rear face of the plug 8 connecting with the radial bore 9 so that the compressed air can flow through each of them sub-sonically. According to some implementations, the same diameter can be selected for both the radial bore 9 and the axial hole 10.

A set of female thread holes 11 open to the rear face of the plug 8 are provided according to the usual practice in mechanical engineering to fix a connector part 12 to plug 8. This connector part 12 is provided with a standardized female thread bore 13 where a standard fast connector 14 is provided to provide a fluid connection with a flexible pipeline 15 as shown in FIG. 6. According to some implementations, the liquid inlet 6 is drilled into the front face of the connector part 12 to enable the flow of liquid from the flexible pipeline 15 to the rear sub-chamber 1b. In such an implementation, a front face 12a of the connector part 12 partially delimits the rear sub-chamber 1b acting as a rear wall of the rear sub-chamber. As shown in FIG. 1, the connector part 12 may include an air conduit 17 that is in fluid communication with air passage 10. According to some implementations the connector part 12 is a monolithic structure that includes both a liquid conduit and an air/gas conduit. An O-ring or gasket 16 disposed between the connector part 12 and the plug 8 provides an air-tight seal between them.

With continued reference to FIG. 6, according to some implementations the housing 7 is provided with a thread hole 71 at its rear face where an external connector fitting 19 is screwed providing a fluid connection to a radially outer-most conduit 31 of a coaxial hose 30. The flexible pipeline 15 passes through a hole 191 in the external connector fitting 19 and constitutes the radially innermost conduit of the coaxial hose 30. Liquid is supplied through the flexible pipeline 15 while compressed air is supplied through the clearance between the flexible pipeline 15 and the outer-most hose 31 or the hole 191. The total cross section of such a clearance between the flexible pipeline 15 and the outer-most conduit 31 or the hole 191 is preferably large enough to cause the compressed air to flow at a subsonic speed with a low pressure drop. The coaxial hose 30 is flexible and long enough to enable the user to manipulate the housing 7 to aim the outlet of the nozzle towards any desired decontamination or disinfecting location.

According to some implementations a "T" fitting 32 is provided to merge a liquid supply line 33 and a compressed air supply line 34. The flexible pipeline 15 passes through a hole 351 in an external connector fitting 35 connected to a first branch 321 of the "T" fitting 32, to finally be connected with a standard fast connector 36 screwed internally to the thread of a second branch 322 of the "T" fitting 32. A liquid connector fitting 37 screwed externally to the thread of the second branch 322 of the "T" fitting 32 is connected to the liquid supply line 33 arranged so that liquid is supplied to the flexible pipeline 15. Additionally, a compressed air connector fitting 38 is connected to a third branch 323 of the "T" fitting 32 to provide compressed air from the compressed air supply line 34.

It is important to note that the functioning of the nozzle 100 is in no way limited to a liquid and gas delivery system as disclosed in the preceding three paragraphs. Any gas and liquid delivery system can be used in conjunction with the nozzle 100, so long as an achievement of the first, second and third atomization stages as disclosed herein is capable of being met when the nozzle 100 is operated in the first mode.

Exemplary implementations have been disclosed and described herein. It is to be appreciated however, that the present invention is in no way to be construed as to being limited to these examples.

## Claims

1. A nozzle assembly comprising:
a cylindrical outlet conduit (4) that exhaust to the environment; the diameter of the outlet conduit (4) being equal to or less than one-half the length of the outlet conduit (4);
a rear sub-chamber (1b);
a liquid inlet (6) that opens into the rear sub-chamber (1b);
a front sub-chamber (1a) in fluid communication with both the outlet conduit (4) and the rear sub-chamber (1b);
a cylindrical air inlet conduit (3), the diameter of the air inlet conduit (3) being equal to or less than one-half the length of the air inlet conduit (3), configured to deliver a pressurized supersonic air stream into the front sub-chamber (1a), the air inlet conduit (3) having an outlet that faces a wall (24) of the front sub-chamber (1a), and
a dividing member (2) disposed between the front and rear sub-chambers (1a and 1b), the dividing member (2) including at least first and second fluid passages (21a, 21b), the first fluid passage (21a) being configured essentially opposed to the said air inlet conduit (3), therefore to transport air from the front sub-chamber (1a) back to the rear sub-chamber (1b), the second fluid passage (21b) being configured apart from the first fluid passage (21a) to transport a first mist comprising an air and liquid mixture from the rear sub-chamber (1b) to the front sub-chamber (1a);
the air inlet conduit (3), the front sub-chamber (1a) and the dividing member (2) being configured such that upon pressurized air being introduced into the air inlet conduit (3) at a pressure higher than 4 bar and its supersonic critical pressure, the generated pressurized supersonic air stream strikes the wall (24) of the front sub-chamber (1a) and is divided into first and second air streams that flow in respective first and second directions, the first direction being towards the outlet conduit (4), the second direction being towards the first fluid passage (21a) of the dividing member (2), the first fluid passage (21a) configured to permit air in the second air stream to proceed into the rear sub-chamber (1b).

2. The nozzle assembly according to claim 1, wherein the outlet conduit (4), the rear sub-chamber (1b), the liquid inlet (6), the front sub-chamber (1a), the dividing member (2), and the air inlet conduit (3) are configured such that upon the pressurized air being introduced into the air inlet conduit (3) and a liquid is introduced into the liquid inlet (6) at a pressure low enough to prevent the liquid from filling up the rear subchamber (1b) and a first atomization of the liquid creating the first mist occurs in the rear sub-chamber (1b) by the air proceeding into the rear sub-chamber (1b), the cited mist flowing through the second fluid passage (21b) of the dividing member (2) into the front sub-chamber (1a).

3. The nozzle assembly according to claim 2, wherein the the second fluid passage (21b) of the dividing member (2) leads into the pressurized supersonic air stream in the front sub-chamber (1a) causing a second atomization of the liquid in the first mist to create a second mist inside the front sub-chamber (1a).

4. The nozzle assembly according to claim 3, wherein the outlet conduit (4), the rear sub-chamber (1b), the liquid inlet (6), the front sub-chamber (1a), the dividing member (2), and the air inlet conduit (3) are configured such that after the pressurized air is introduced into the air inlet conduit (3) and the liquid is introduced into the liquid inlet (6), pressure in the front sub-chamber (1a) remains over the critical value for such configuration so that the second mist produced in the front sub-chamber (1a) is accelerated to supersonic speeds in the nozzle outlet conduit (4).

5. The nozzle according to claim 1, wherein the air inlet conduit (3) has a first cross-sectional area and the outlet conduit (4) has a second cross-sectional area, the second cross-sectional area being greater than the first cross-sectional area.

6. The nozzle according to claim 1, wherein the first and second fluid passages (21a, 21b) of the dividing member (2) respectively comprise a first axial groove and a second axial groove formed in an outer wall of the dividing member (2).

7. The nozzle according to claim 1, wherein an entirety of all of the first and second fluid passages (21a, 21b) are straight.

8. The nozzle according to claim 7, wherein all of the first and second fluid passages (21a, 21b) are arranged parallel to the wall of the front sub-chamber (1a) onto which the generated pressurized supersonic air stream strikes.

9. The nozzle according to claim 1, wherein the first and second fluid passages (21a, 21b) of the dividing member (2) respectively comprise a first axial through hole (21c) and a second axial through hole (21d).

10. The nozzle assembly according to claim 1, wherein the dividing member (2) is in the form of a disk.

11. The nozzle assembly according to claim 1, wherein the dividing member (2) is capable of assuming multiple axial positions for the purpose of altering the volume of the front and rear sub-chambers (1a, 1b).

12. The nozzle assembly according to claim 1, further comprising a radial bore (9) through which the pressurized air is delivered to the air inlet conduit (3), the radial bore (9) having a first diameter, the air inlet conduit (3) having a second diameter less than the first diameter.

13. The nozzle assembly according to claim 1, wherein the front sub-chamber (1a) is delimited by a back face of a plug (8), the outlet conduit (4) extending through a portion of the plug (8).

14. The nozzle assembly according to claim 13, further comprising a handle (7) that can be manipulated by a user, the plug (8) being attached to a front end of the handle (7), a front face of the plug (8) and the front end of the handle being flush with one another.

15. The nozzle assembly according to claim 14, wherein the plug (8) and handle (7) are attached by a threaded connection.

16. The nozzle assembly according to claim 13, wherein the outlet conduit (4) is disposed between a cone-shaped hole and a conic outlet mouth (5) that are each formed in the plug (8), each of the cone-shaped hole and the conic outlet mouth (5) having a central axis that are axially aligned.

17. The nozzle assembly according to claim 1, further comprising a connector part (12) through which the pressurized air and liquid are supplied, the connector part (12) having a first conduit (13) in fluid communication with the liquid inlet (6) and a second conduit (17) in fluid communication with the air inlet conduit (3).

18. The nozzle assembly according to claim 17, wherein the connector part (12) is a monolithic structure.

19. The nozzle assembly according to claim 17, comprising the connector part (12) through which the pressurized air and liquid are supplied, the connector part (12) having the first conduit (13) in fluid communication with the liquid inlet (6) and the second conduit (17) in fluid communication with the air inlet conduit (3), the connector part (12) being coupled to the plug by a fast connector (14).

20. The nozzle assembly according to claim 17, further comprising a flexible coaxial hose coupled to the connector part (12), the coaxial hose (30) having an inner conduit (15) and an outer conduit (31) surrounding the inner conduit (15), one of the inner and outer conduits (15, 31) being in fluid communication with the first conduit (13) of the connector part (12), the other of the inner and outer conduits (15, 31) being in fluid communication with the second conduit (17) of the connector part (12).

## Patentansprüche

1. Düsenanordnung, umfassend:
eine zylindrische Auslassleitung (4), die in die Umgebung mündet, wobei der Durchmesser der Auslassleitung (4) gleich oder kleiner als die Hälfte der Länge der Auslassleitung (4) ist;
eine hintere Teilkammer (1b);
einen Flüssigkeitseinlass (6), der in die hintere Teilkammer (1b) mündet;
eine vordere Teilkammer (1a), die sowohl mit der Auslassleitung (4) als auch mit der hinteren Teilkammer (1b) in Fluidverbindung steht;
eine zylindrische Lufteinlassleitung (3), wobei der Durchmesser der Lufteinlassleitung (3) gleich oder kleiner als die Hälfte der Länge der Lufteinlassleitung (3) ist, die dazu ausgebildet ist, einen unter Druck stehenden Überschall-Luftstrom in die vordere Teilkammer (1a) zu leiten, wobei die Lufteinlassleitung (3) einen Auslass aufweist, der einer Wand (24) der vorderen Teilkammer (1a) zugewandt ist, und
ein Trennelement (2), das zwischen der vorderen und der hinteren Teilkammer (1a und 1b) angeordnet ist, wobei das Trennelement (2) mindestens einen ersten und einen zweiten Fluidkanal (21a, 21b) umfasst, wobei der erste Fluidkanal (21a) im Wesentlichen der genannten Lufteinlassleitung (3) gegenüberliegend angeordnet ist, um somit Luft aus der vorderen Teilkammer (1a) zurück in die hintere Teilkammer (1b) zu leiten, wobei der zweite Fluidkanal (21b) getrennt vom ersten Fluidkanal (21a) angeordnet ist, um einen ersten Nebel, der ein Luft-Flüssigkeits-Gemisch umfasst, aus der hinteren Teilkammer (1b) in die vordere Teilkammer (1a) zu leiten;
wobei die Lufteinlassleitung (3), die vordere Teilkammer (1a) und das Trennelement (2) so ausgebildet sind, dass bei Einleitung von Druckluft in die Lufteinlassleitung (3) mit einem Druck, der höher als 4 bar und höher als ihr kritischer Überschalldruck ist, der erzeugte unter Druck stehende Überschall-Luftstrom auf die Wand (24) der vorderen Teilkammer (1a) trifft und in einen ersten und einen zweiten Luftstrom aufgeteilt wird, die in eine jeweilige erste und zweite Richtung strömen, wobei die erste Richtung zur Auslassleitung (4) hin und die zweite Richtung zum ersten Fluidkanal (21a) des Trennelements (2) hin verläuft, wobei der erste Fluidkanal (21a) so ausgebildet ist, dass Luft im zweiten Luftstrom in die hintere Teilkammer (1b) gelangen kann.

2. Düsenanordnung nach Anspruch 1, wobei die Auslassleitung (4), die hintere Teilkammer (1b), der Flüssigkeitseinlass (6), die vordere Teilkammer (1a), das Trennelement (2) und die Lufteinlassleitung (3) so ausgebildet sind, dass bei Einleiten der Druckluft in die Lufteinlassleitung (3) und bei Einleiten einer Flüssigkeit in den Flüssigkeitseinlass (6) mit einem Druck, der niedrig genug ist, um zu verhindern, dass die Flüssigkeit die hintere Teilkammer (1b) füllt, eine erste Zerstäubung der Flüssigkeit, die den ersten Nebel erzeugt, in der hinteren Teilkammer (1b) durch die in die hintere Teilkammer (1b) strömende Luft erfolgt, wobei der genannte Nebel durch den zweiten Fluidkanal (21b) des Trennelements (2) in die vordere Teilkammer (1a) strömt.

3. Düsenanordnung nach Anspruch 2, wobei der zweite Fluidkanal (21b) des Trennelements (2) in den unter Druck stehenden Überschall-Luftstrom in der vorderen Teilkammer (1a) mündet, wodurch eine zweite Zerstäubung der Flüssigkeit im ersten Nebel bewirkt wird, um einen zweiten Nebel innerhalb der vorderen Teilkammer (1a) zu erzeugen.

4. Düsenanordnung nach Anspruch 3, wobei die Auslassleitung (4), die hintere Teilkammer (1b), der Flüssigkeitseinlass (6), die vordere Teilkammer (1a), das Trennelement (2) und die Lufteinlassleitung (3) so ausgebildet sind, dass nach dem Einleiten der Druckluft in die Lufteinlassleitung (3) und dem Einleiten der Flüssigkeit in den Flüssigkeitseinlass (6) der Druck in der vorderen Teilkammer (1a) über dem für diese Konfiguration kritischen Wert bleibt, sodass der in der vorderen Teilkammer (1a) erzeugte zweite Nebel in der Düsenauslassleitung (4) auf Überschallgeschwindigkeit beschleunigt wird.

5. Düse nach Anspruch 1, wobei die Lufteinlassleitung (3) eine erste Querschnittsfläche und die Auslassleitung (4) eine zweite Querschnittsfläche aufweist, wobei die zweite Querschnittsfläche größer ist als die erste Querschnittsfläche.

6. Düse nach Anspruch 1, wobei der erste und der zweite Fluidkanal (21a, 21b) des Trennelements (2) jeweils eine erste axiale Nut und eine zweite axiale Nut umfassen, die in einer Außenwand des Trennelements (2) ausgebildet sind.

7. Düse nach Anspruch 1, wobei alle ersten und zweiten Fluidkanäle (21a, 21b) durchgehend gerade verlaufen.

8. Düse nach Anspruch 7, wobei alle ersten und zweiten Fluidkanäle (21a, 21b) parallel zu der Wand der vorderen Teilkammer (1a) angeordnet sind, auf die der erzeugte unter Druck stehende Überschall-Luftstrom auftrifft.

9. Düse nach Anspruch 1, wobei der erste und der zweite Fluidkanal (21a, 21b) des Trennelements (2) jeweils eine erste axiale Durchgangsbohrung (21c) und eine zweite axiale Durchgangsbohrung (21d) umfassen.

10. Düsenanordnung nach Anspruch 1, wobei das Trennelement (2) die Form einer Scheibe aufweist.

11. Düsenanordnung nach Anspruch 1, wobei das Trennelement (2) mehrere axiale Positionen einnehmen kann, um das Volumen der vorderen und der hinteren Teilkammer (1a, 1b) zu verändern.

12. Düsenanordnung nach Anspruch 1, ferner umfassend eine radiale Bohrung (9), durch die die Druckluft zur Lufteinlassleitung (3) geleitet wird, wobei die radiale Bohrung (9) einen ersten Durchmesser aufweist und die Lufteinlassleitung (3) einen zweiten Durchmesser aufweist, der kleiner ist als der erste Durchmesser.

13. Düsenanordnung nach Anspruch 1, wobei die vordere Teilkammer (1a) durch eine Rückseite eines Stopfens (8) begrenzt ist, wobei sich die Auslassleitung (4) durch einen Abschnitt des Stopfens (8) erstreckt.

14. Düsenanordnung nach Anspruch 13, ferner umfassend einen Griff (7), der von einem Benutzer gehandhabt werden kann, wobei der Stopfen (8) an einem vorderen Ende des Griffs (7) befestigt ist und eine Vorderseite des Stopfens (8) sowie das vordere Ende des Griffs bündig miteinander abschließen.

15. Düsenanordnung nach Anspruch 14, wobei der Stopfen (8) und der Griff (7) durch eine Schraubverbindung befestigt sind.

16. Düsenanordnung nach Anspruch 13, wobei die Auslassleitung (4) zwischen einer kegelförmigen Bohrung und einer konischen Auslassöffnung (5) angeordnet ist, die jeweils im Stopfen (8) ausgebildet sind, wobei die kegelförmige Bohrung und die konische Auslassöffnung (5) jeweils eine Mittelachse aufweisen und diese Mittelachsen axial fluchten.

17. Düsenanordnung nach Anspruch 1, ferner umfassend ein Anschlussteil (12), durch das die Druckluft und die Flüssigkeit zugeführt werden, wobei das Anschlussteil (12) einen ersten Kanal (13) in Fluidverbindung mit dem Flüssigkeitseinlass (6) und einen zweiten Kanal (17) in Fluidverbindung mit der Lufteinlassleitung (3) aufweist.

18. Düsenanordnung nach Anspruch 17, wobei das Anschlussteil (12) eine monolithische Struktur ist.

19. Düsenanordnung nach Anspruch 17, umfassend das Anschlussteil (12), durch das die Druckluft und die Flüssigkeit zugeführt werden, wobei das Anschlussteil (12) den ersten Kanal (13) in Fluidverbindung mit dem Flüssigkeitseinlass (6) und den zweiten Kanal (17) in Fluidverbindung mit der Lufteinlassleitung (3) aufweist, wobei das Anschlussteil (12) über eine Schnellkupplung (14) mit dem Stopfen verbunden ist.

20. Düsenanordnung nach Anspruch 17, ferner umfassend einen flexiblen Koaxialschlauch, der mit dem Anschlussteil (12) verbunden ist, wobei der Koaxialschlauch (30) einen inneren Kanal (15) und einen den inneren Kanal (15) umgebenden äußeren Kanal (31) aufweist, wobei einer der inneren und äußeren Kanäle (15, 31) in Fluidverbindung mit dem ersten Kanal (13) des Anschlussteils (12) steht und der jeweils andere der inneren und äußeren Kanäle (15, 31) in Fluidverbindung mit dem zweiten Kanal (17) des Anschlussteils (12) steht.

## Revendications

1. Ensemble de buse comprenant :
un conduit de sortie cylindrique (4) débouchant à l'environnement, le diamètre du conduit de sortie (4) étant inférieur ou égal à la moitié de la longueur du conduit de sortie (4) ;
une sous-chambre arrière (1b) ;
une entrée de liquide (6) qui débouche dans la sous-chambre arrière (1b) ;
une sous-chambre avant (1a) en communication fluidique à la fois avec le conduit de sortie (4) et avec la sous-chambre arrière (1b) ;
un conduit d'entrée d'air cylindrique (3), le diamètre du conduit d'entrée d'air (3) étant inférieur ou égal à la moitié de la longueur du conduit d'entrée d'air (3), configuré pour délivrer un flux d'air supersonique sous pression dans la sous-chambre avant (1a), le conduit d'entrée d'air (3) présentant une sortie qui fait face à une paroi (24) de la sous-chambre avant (1a), et
un élément de séparation (2) disposé entre les sous-chambres avant et arrière (1a et 1b), l'élément de séparation (2) comprenant au moins un premier et un deuxième passage de fluide (21a, 21b), le premier passage de fluide (21a) étant configuré de manière essentiellement opposée audit conduit d'entrée d'air (3), afin de transporter ainsi de l'air depuis la sous-chambre avant (1a) en retour vers la sous-chambre arrière (1b), le deuxième passage de fluide (21b) étant configuré à l'écart du premier passage de fluide (21a) afin de transporter une première brume comprenant un mélange d'air et de liquide depuis la sous-chambre arrière (1b) vers la sous-chambre avant (1a) ;
le conduit d'entrée d'air (3), la sous-chambre avant (1a) et l'élément de séparation (2) étant configurés de telle sorte que, lorsque de l'air sous pression est introduit dans le conduit d'entrée d'air (3) à une pression supérieure à 4 bars et supérieure à sa pression critique supersonique, le flux d'air supersonique sous pression généré frappe la paroi (24) de la sous-chambre avant (1a) et est divisé en un premier et un deuxième flux d'air qui s'écoulent respectivement selon une première et une deuxième direction, la première direction étant dirigée vers le conduit de sortie (4), la deuxième direction étant dirigée vers le premier passage de fluide (21a) de l'élément de séparation (2), le premier passage de fluide (21a) étant configuré pour permettre à l'air du deuxième flux d'air de pénétrer dans la sous-chambre arrière (1b).

2. Ensemble de buse selon la revendication 1, dans lequel le conduit de sortie (4), la sous-chambre arrière (1b), l'entrée de liquide (6), la sous-chambre avant (1a), l'élément de séparation (2) et le conduit d'entrée d'air (3) sont configurés de telle sorte que, lorsque l'air sous pression est introduit dans le conduit d'entrée d'air (3) et qu'un liquide est introduit dans l'entrée de liquide (6) à une pression suffisamment basse pour empêcher le liquide de remplir la sous-chambre arrière (1b), une première atomisation du liquide créant la première brume se produit dans la sous-chambre arrière (1b) sous l'effet de l'air pénétrant dans la sous-chambre arrière (1b), ladite brume s'écoulant à travers le deuxième passage de fluide (21b) de l'élément de séparation (2) dans la sous-chambre avant (1a).

3. Ensemble de buse selon la revendication 2, dans lequel le deuxième passage de fluide (21b) de l'élément de séparation (2) débouche dans le flux d'air supersonique sous pression présent dans la sous-chambre avant (1a), provoquant une deuxième atomisation du liquide contenu dans la première brume afin de créer une deuxième brume à l'intérieur de la sous-chambre avant (1a).

4. Ensemble de buse selon la revendication 3, dans lequel le conduit de sortie (4), la sous-chambre arrière (1b), l'entrée de liquide (6), la sous-chambre avant (1a), l'élément de séparation (2) et le conduit d'entrée d'air (3) sont configurés de telle sorte qu'après l'introduction de l'air sous pression dans le conduit d'entrée d'air (3) et l'introduction du liquide dans l'entrée de liquide (6), la pression dans la sous-chambre avant (1a) demeure supérieure à la valeur critique propre à une telle configuration, de sorte que la deuxième brume produite dans la sous-chambre avant (1a) est accélérée jusqu'à des vitesses supersoniques dans le conduit de sortie de buse (4).

5. Buse selon la revendication 1, dans laquelle le conduit d'entrée d'air (3) présente une première aire de section transversale et le conduit de sortie (4) présente une deuxième aire de section transversale, la deuxième aire de section transversale étant supérieure à la première aire de section transversale.

6. Buse selon la revendication 1, dans laquelle les premier et deuxième passages de fluide (21a, 21b) de l'élément de séparation (2) comprennent respectivement une première rainure axiale et une deuxième rainure axiale ménagées dans une paroi extérieure de l'élément de séparation (2).

7. Buse selon la revendication 1, dans laquelle la totalité des premier et deuxième passages de fluide (21a, 21b) est rectiligne.

8. Buse selon la revendication 7, dans laquelle tous les premier et deuxième passages de fluide (21a, 21b) sont disposés parallèlement à la paroi de la sous-chambre avant (1a) que frappe le flux d'air supersonique sous pression généré.

9. Buse selon la revendication 1, dans laquelle les premier et deuxième passages de fluide (21a, 21b) de l'élément de séparation (2) comprennent respectivement un premier trou traversant axial (21c) et un deuxième trou traversant axial (21d).

10. Ensemble de buse selon la revendication 1, dans lequel l'élément de séparation (2) se présente sous la forme d'un disque.

11. Ensemble de buse selon la revendication 1, dans lequel l'élément de séparation (2) est apte à occuper de multiples positions axiales dans le but de modifier le volume des sous-chambres avant et arrière (1a, 1b).

12. Ensemble de buse selon la revendication 1, comprenant en outre un alésage radial (9) à travers lequel l'air sous pression est amené au conduit d'entrée d'air (3), l'alésage radial (9) présentant un premier diamètre, le conduit d'entrée d'air (3) présentant un deuxième diamètre inférieur au premier diamètre.

13. Ensemble de buse selon la revendication 1, dans lequel la sous-chambre avant (1a) est délimitée par une face arrière d'un bouchon (8), le conduit de sortie (4) s'étendant à travers une portion du bouchon (8).

14. Ensemble de buse selon la revendication 13, comprenant en outre une poignée (7) pouvant être manipulée par un utilisateur, le bouchon (8) étant fixé à une extrémité avant de la poignée (7), une face avant du bouchon (8) et l'extrémité avant de la poignée affleurant l'une avec l'autre.

15. Ensemble de buse selon la revendication 14, dans lequel le bouchon (8) et la poignée (7) sont assemblés par un raccord fileté.

16. Ensemble de buse selon la revendication 13, dans lequel le conduit de sortie (4) est disposé entre un trou en forme de cône et une embouchure de sortie conique (5) qui sont chacun ménagés dans le bouchon (8), le trou en forme de cône et l'embouchure de sortie conique (5) présentant chacun un axe central, ces axes étant alignés axialement.

17. Ensemble de buse selon la revendication 1, comprenant en outre une pièce de connexion (12) par laquelle l'air sous pression et le liquide sont amenés, la pièce de connexion (12) présentant un premier conduit (13) en communication fluidique avec l'entrée de liquide (6) et un deuxième conduit (17) en communication fluidique avec le conduit d'entrée d'air (3).

18. Ensemble de buse selon la revendication 17, dans lequel la pièce de connexion (12) est une structure monobloc.

19. Ensemble de buse selon la revendication 17, comprenant la pièce de connexion (12) par laquelle l'air sous pression et le liquide sont amenés, la pièce de connexion (12) présentant le premier conduit (13) en communication fluidique avec l'entrée de liquide (6) et le deuxième conduit (17) en communication fluidique avec le conduit d'entrée d'air (3), la pièce de connexion (12) étant accouplée au bouchon par un connecteur rapide (14).

20. Ensemble de buse selon la revendication 17, comprenant en outre un tuyau coaxial souple accouplé à la pièce de connexion (12), le tuyau coaxial (30) présentant un conduit intérieur (15) et un conduit extérieur (31) entourant le conduit intérieur (15), l'un des conduits intérieur et extérieur (15, 31) étant en communication fluidique avec le premier conduit (13) de la pièce de connexion (12), l'autre des conduits intérieur et extérieur (15, 31) étant en communication fluidique avec le deuxième conduit (17) de la pièce de connexion (12).
